# FASCICULE DE BREVET EUROPEEN

(11) **EP 1 237 562 B1**
(45) Date de publication et mention de la délivrance du brevet: **10.11.2004**
(21) Numéro de dépôt: 00985359.9
(22) Date de dépôt: 30.11.2000
(51) Int. Cl.: A61K 35/78, A61P 35/00, A61P 31/12, A61P 33/02, A61P 33/06

(54) **PROCEDE DE PREPARATION D'EXTRAITS DE MIKANIA CONTENANT DU MIKANOLIDE ET DU DIHYDROMIKANOLIDE ET UTILISATION DANS LE TRAITEMENT DES MALADIES PROLIFERATIVES**
VERFAHREN ZUR HERSTELLUNG VON MIKANIA EXTRAKTEN ENTHALTEND MIKANOLIDE UND DIHYDROMIKANOLIDE UND VERWENDUNG IN DER BEHANDLUNG PROLIFERATIVER ERKRANKUNGEN
PROCESS FOR PREPARING MIKANIA EXTRACTS CONTAINING MIKANOLIDE AND DIHYDROMIKANOLIDE AND USE IN THE TREATMENT OF PROLIFERATIVE DISORDERS

(30) Priorité: 01.12.1999 FR 9915126
(43) Date de publication de la demande: 11.09.2002
(73) Titulaire: SOCIETE DE CONSEILS DE RECHERCHES ET D'APPLICATIONS SCIENTIFIQUES (S.C.R.A.S.), 75016 Paris (FR)
(72) Inventeur: TENG, Beng, Poon, F-91190 Gif-sur-Yvette (FR); PREVOST, Grégoire, F-92160 Antony (FR); BREZAK PANNETIER, Marie-Christine, F-92160 Antony (FR); MOUMEN, Mohamed, F-91700 Sainte-Genevieve-des-Bois (FR)
(74) Mandataire: Bourgouin, André
(86) Numéro de dépôt international: PCT/FR2000/003337
(87) Numéro de publication internationale: WO 2001/039720

(56) Documents cités:
- PETREA C. FACEY ET AL.: "INVESTIGATION OF EXTRACTS FROM 25 JAMAICAN FOLKLORE PLANTS FOR ANTIBACTERIAL ACTIVITY: STRUCTURE-ACTIVITY RELATIONSHIP OF MIKANOLIDE AND ITS DERIVATIVES" ABSTRACTS OF PAPERS OF THE NATIONAL MEETING OF THE AMERICAN CHEMICAL SOCIETY, vol. 218, 22 - 26 août 1999, page medi 294 XP000929986 XX, XX
- M. AHMED: "FURTHER DILACTONES FROM MIKANIA CORDATA" PHARMAZIE., vol. 45, septembre 1990 (1990-09), page 697 XP002145554 VEB VERLAG VOLK UND GESUNDHEIT. BERLIN., DD ISSN: 0031-7144
- FACEY P C ET AL: "INVESTIGATION OF PLANTS USED IN JAMAICAN FOLK MEDICINE FOR ANTI-BACTERIAL ACTIVITY" JOURNAL OF PHARMACY AND PHARMACOLOGY,LONDON,GB, vol. 51, 1999, pages 1455-1460, XP000929990 ISSN: 0022-3573
- GRACIELA ZAMORANO ET AL.: "SESQUITERPENE DILACTONES FROM MIKANIA YPACARAYENSIS" PHYTOCHEMISTRY., vol. 38, 1995, pages 1257-1260, XP000929993 PERGAMON PRESS., GB ISSN: 0031-9422

## Description

La présente invention concerne, en tant que médicaments et notamment comme agents anti-prolifératifs des extraits enrichis en dihydromikanolide. Elle a aussi pour objet leur obtention par extraction de la plante *Mikania micrantha* par un procédé décrit ci-après.

Depuis longtemps, les plantes sont une source de produits pharmaceutiques et d'ingrédients de la médicine traditionnelle (Phillipson, J.D., *Trans. R. Soc*. *Trop. Med*. *Hyg*. (1994), **88 Suppl. 1,** S17-S19). Les médicaments formés par des produits naturels ou leurs analogues synthétiques jouent un rôle très important.

Par exemple, en oncologie, l'on trouve des produits comme le taxol (Paclitaxel®), la vincristine (Oncovin®), la vinorelbine (Navelbine®, le téniposide (Vumon®), et plusieurs analogues de la camptothécine (CPT) solubles dans l'eau (Pezzuto, J.M., *Biochem. Pharmacol*. (1997), **53**, 121-133). D'autres produits issus de plantes sont par ailleurs à l'étude pour la prévention de la resténose.

Dans le domaine du traitement du cancer, par exemple, la camptothécine est un alkaloïde pentacyclique isolé du bois de *Camptotheca acuminata* (Wall, M.E., *Med. Res. Rev*. (1998), **18**, 299-314). Initialement, la CPT a été identifiée comme un inhibiteur de la prolifération tumorale dans des modèles de cellules humaines. Par la suite, le mécanisme d'action de la CPT a été identifié comme un poison de l'enzyme topoisomérase I impliquée dans la réplication de l'ADN. Un nombre croissant d'analogues de la CPT (topotécan, CPT-11, BN-80915) sont utilisés en traitement anticancéreux chez l'homme (Lavergne, O. et coll., *J*. *Med*. *Chem*. (1998), **41**, 5410-5419).

Un autre exemple est le taxol, un alkaloïde terpénique isolé de *Taxus brevifolia*. Le taxol est un cytotoxique puissant qui inhibe la tubuline, une protéine impliquée dans la mitose. C'est un produit particulièrement efficace dans les cancers de l'ovaire.

Par ailleurs, les alkaloïdes *vinca* ont d'abord été extraits de *Catharanthus roseus*. Ils étaient d'abord utilisés ethnobotaniquement pour d'autres indications que le traitement du cancer.

En ce qui concerne la prévention de la resténose, des études indiquent qu'elle pourrait être obtenue grâce à une protéine issue de *Saponaria officinalis*, la saponine, recombinée au facteur de croissance du fibroblaste (FGF pour *Fibroblast Growth Factor*). Ce dernier permettrait le ciblage des cellules vasculaires du fait de son affinité pour le récepteur à FGF (Lin PH et coll., *Atherosclerosis* (avril 1998), **137(2)**, 277-289).

La méthode pour découvrir de tels produits est un fractionnement des molécules de l'extrait de plante suivi de l'évaluation de leurs activités biologiques. Mais en regard du nombre de genres, d'espèces et de variétés de plantes, et de la quantité impressionnante de molécules issues de ces plantes, l'identification d'une molécule et de son activité biologique reste une entreprise très difficile à mener.

*Mikania micrantha* est une herbe pandémique dans les régions tropicales. Elle prolifère de façon extrêmement rapide, à tel point qu'elle est surnommée "*a-mile-a-minute*", c'est-à-dire un mile par minute. *Mikania scandens* et *cordata* sont souvent confondues avec elle. Ces trois espèces contiennent notamment du mikanolide **(I)**, du dihydromikanolide **(II)**, du scandénolide **(III)** et du dihydroscandénolide **(IV)**.

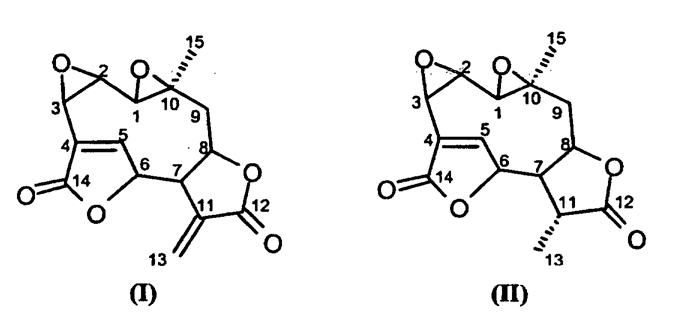

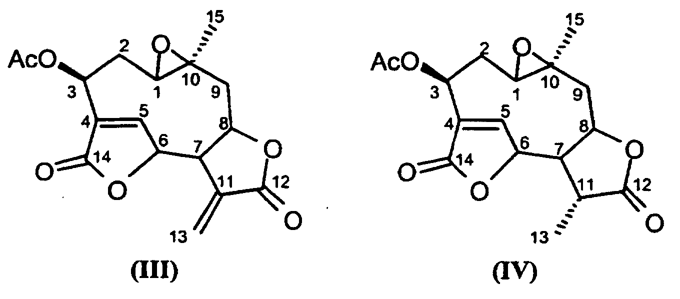

### Structures du mikanolide, du dihydromikanolide, du scandénolide et du dihydroscandénolide

L'extraction du mikanolide, du dihydromikanolide, du scandénolide et du dihydroscandénolide à partir de *Mikania scandens, Mikania cordata* et *Mikania micrantha* a été décrite dans les références suivantes respectivement : Herz, *Tetrahedron Lett.* (1967), **32**, 3111-15 ; Kiang, *Phytochemistry* (1968), **7(6)**, 1035-7 ; et Cuenca, *J. Nat. Prod.* (1988), **51(3)**, 625-6. Les solvants utilisés comprennent le chloroforme et le diéthyléther qui ne sont pas utilisables de façon industrielle du fait de leur toxicité ou de leur inflammabilité.

Ethnobotaniquement, *Mikania scandens* et *cordata* ont été et sont encore utilisées par la médicine traditionnelle comme agent anti-asthmatique, analgésique, anti-rhumatismal, anti-inflammatoire, anti-cancéreux, fébrifuge et vermifuge en Amérique centrale où son nom commun est le guaco. C'est seulement dans la dernière décennie que certaines de ces propriétés ont été étudiées scientifiquement, en particulier les extraits de *Mikania cordata* dans le domaine de l'inflammation.

Récemment, la demanderesse a testé l'activité anti-proliférative et trouvé que, de façon surprenante, un extrait enrichi en dihydromikanolide et mikanolide ou ces derniers purs présentent une puissante activité. De plus, le dihydromikanolide et le mikanolide inhibent la réplication de l'ADN en inhibant les enzymes ADN polymérases nécessaire pour la multiplication des cellules eucaryotes et procaryotes ainsi que des virus.

Selon l'invention, ledit extrait, obtenu à partir de feuilles de *Mikania micrantha, Mikania scandens* et *Mikania cordata*, comporte plus de 50%, et de préférence plus de 60 % de mikanolide et dihydromikanolide. Encore plus préférentiellement, ledit extrait comporte plus de 70 % de mikanolide et dihydromikanolide. De préférence, ledit extrait comporte par ailleurs moins de 10%, et plus préférentiellement moins de 5%, de scandénolide et de dihydroscandénolide. Par ailleurs, on préférera également les extraits comportant plus de 20% de dihydromikanolide, et plus préférentiellement, plus de 30% de dihydromikanolide.

Ledit extrait peut être préparé selon la méthode d'extraction décrite ci-après à partir de *Mikania micrantha*, les espèces *Mikania scandens* et *Mikania cordata*, n'étant toutefois pas éliminées.

L'invention concerne donc tout d'abord un procédé de préparation d'un extrait selon l'invention, caractérisé en ce qu'il comporte les étapes successives suivantes :
- broyage et séchage de feuilles de *Mikania micrantha, Mikania scandens* et *Mikania cordata* ;
- addition, aux feuilles broyées de *Mikania micrantha, Mikania scandens* et *Mikania cordata*, d'un solvant choisi parmi le toluène, l'acétate d'éthyle, des mélanges de toluène et d'acétate d'éthyle, des mélanges de toluène et d'acétone, des mélanges d'acétate d'éthyle et d'heptane, et des mélanges d'heptane et d'acétone dans des proportions de 7:3 à 3:7 ;
- filtration et récupération du filtrat ;
- concentration de l'extrait de la solution d'extrait primaire pour obtenir une concentration de 2,5 à 10% en extrait sec ;
- partition de l'extrait concentré obtenu à l'étape précédente entre le solvant d'extraction et un mélange comprenant de 10% à 50% de méthanol ou d'éthanol dans l'eau ;
- lavage de la phase alcool-eau avec du n-hexane ou de l'heptane ;
- élimination de l'alcool de la phase alcool-eau ;
- extraction de la phase aqueuse obtenue avec de l'acétate d'éthyle ;
- séchage de la phase acétate d'éthyle avec un agent desséchant ;
- évaporation des solvants et récupération de l'extrait sec ;
- purification de l'extrait sec obtenu par une méthode de séparation physico-chimique ; et
- obtention, après séchage, d'un extrait sec selon l'invention.

Selon l'invention, la méthode de séparation physico-chimique peut être, par exemple, une filtration sur gel de silice, ou bien encore, selon une variante préférée, une cristallisation dans le méthanol, l'éthanol ou l'acétone.

Si l'on choisit la filtration sur gel de silice, les éluants employés seront de préférence des mélanges heptane-acétate d'éthyle de proportions comprises entre 9:1 et 1:1.

Dans le cas où l'on opte pour la cristallisation, celle-ci sera de préférence effectuée à une température comprise entre -10 et 5 °C pendant une durée de préférence comprise entre 2 et 16 heures.

Par ailleurs, selon une variante préférée du procédé ci-dessus, la partition de l'extrait concentré est réalisée à l'aide d'un mélange comprenant de 25 à 30% de méthanol dans l'eau. De préférence, la phase aqueuse inférieure sera chauffée pour faciliter la séparation de phases, par exemple à une température de 30 à 50 °C.

L'extrait sec tel qu'obtenu par le procédé ci-dessus peut être enrichi en dihydromikanolide par conversion du mikanolide en dihydromikanolide par hydrogénation catalytique. Par la suite, une re-cristallisation permettra d'obtenir le composé pur.

L'invention a donc également pour objet un procédé de préparation d'un extrait enrichi en dihydromikanolide caractérisé en ce qu'il consiste en l'hydrogénation de l'extrait sec précédemment décrit, dissous dans de l'acétate d'éthyle, à une température comprise entre 10 et 35 °C, en présence d'un catalyseur d'hydrogénation sous une pression de 1 à 2 atmosphères d'hydrogène pendant une durée de préférence comprise entre 4 et 16 heures. De préférence, le catalyseur d'hydrogénation sera Pd/CaCO₃.

L'invention concerne de plus un extrait sec fortement enrichi en dihydromikanolide tel qu'obtenu par le procédé d'hydrogénation précédemment décrit suivi d'une évaporation des solvants de réaction après l'élimination du catalyseur par filtration, ledit extrait sec étant caractérisé en ce qu'il comprend au moins 50% de dihydromikanolide, et de préférence au moins 92% de dihydromikanolide (qualité pharmaceutique).

Selon l'invention, après hydrogénation, on peut obtenir du dihydromikanolide directement par ajout d'un n-alcane liquide à la solution dans l'acétate d'éthyle de l'extrait enrichi en dihydromikanolide et récupération dudit dihydromikanolide par filtration. De préférence, le n-alcane employé pour obtenir la cristallisation sera du n-hexane ou du n-heptane, et plus préférentiellement du n-heptane.

Le procédé décrit ci-dessus présente l'avantage de pouvoir obtenir du dihydromikanolide à un degré de pureté supérieur à 92%, sans avoir besoin de recourir nécessairement à une séparation de type chromatographique.

Les différents procédés décrits ci-dessus ainsi que d'autres variantes possibles seront décrits en détail dans la partie intitulée "Préparation des produits de l'invention".

La demanderesse vient donc de trouver que les différents extraits de l'invention, possèdent des propriétés pharmacologiques intéressantes, en particulier en tant qu'agents anti-prolifératifs (et notamment en tant qu'agent anti-cancéreux). Ces propriétés sont illustrées dans la partie intitulée "Etude pharmacologique des produits de l'invention".

La présente invention a donc également pour objet, l'utilisation pour la préparation d'un médicament, d'un extrait enrichi en dihydromikanolide tel que décrit précédemment. L'invention a notamment pour objet l'utilisation d'un extrait tel que décrit précédemment pour préparer un médicament destiné à inhiber les ADN polymérases. En particulier, l'invention a pour objet l'utilisation d'un extrait tel que décrit précédemment, pour préparer un médicament destiné à traiter les maladies prolifératives, et en particulier le cancer

Pour les utilisations selon l'invention, on préférera un extrait enrichi en dihydromikanolide.

La composition pharmaceutique préparée selon l'invention peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant du dihydromikanolide peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour un médicament selon l'invention est comprise entre 0,1 mg à 10 g suivant le type de composé actif utilisé.

### Préparation des produits de l'invention

Les solvants suivants peuvent être employés pour l'extraction primaire :
- groupe I : le toluène, l'acétate d'éthyle, des mélanges de toluène et d'acétate d'éthyle, des mélanges de toluène et d'acétone, des mélanges d'acétate d'éthyle et d'heptane, et des mélanges d'heptane et d'acétone dans des proportions de 7:3 à 3:7 et de préférence en mélange 1:1 (proportions exprimées en volume pour volume) ;
- groupe II : l'acétone, l'éthanol et le méthanol ;
- groupe III : des mélanges d'acétone et d'eau, d'éthanol et d'eau ou de méthanol et d'eau dans des proportions de 9:1 à 1:1 (proportions exprimées en volume pour volume, comme pour toute la suite du présent texte, sauf indication contraire).

De préférence, le solvant employé dans la première phase d'extraction sera l'acétate d'éthyle.

Selon l'invention, l'extrait primaire est partitionné dans un mélange comprenant de 10% à 50% de méthanol ou d'éthanol dans l'eau, et de préférence dans un mélange comprenant de 25% à 40% de méthanol dans l'eau à une température de préférence comprise entre 15 et 50°C. De préférence, on chauffera la phase aqueuse inférieure, par exemple à une température comprise entre 30 et 50 °C, ceci afin d'obtenir une séparation de phases plus nette.

Dans le cas où l'extraction primaire est effectuée à l'aide d'un solvant ou d'un mélange de solvants du groupe I, la partition est précédée d'une concentration de la solution d'extrait primaire pour obtenir une concentration de 2,5 à 10% en extrait sec.

Dans le cas où l'extraction primaire est effectuée à l'aide d'un solvant du groupe II, on procède, préalablement à la partition de l'extrait primaire, à une concentration de la solution d'extrait primaire pour obtenir une concentration d'environ 50% en extrait sec suivie d'une dissolution de l'extrait concentré pour obtenir un extrait dilué contenant de 2,5 à 10% d'extrait sec, ladite dissolution étant effectuée à l'aide d'un solvant ou d'un mélange de solvants du groupe I, et de préférence dans l'acétate d'éthyle.

Dans le cas où l'extraction primaire est effectuée à l'aide d'un solvant du groupe III, on procède, préalablement à la partition de l'extrait primaire, à une évaporation de la solution d'extrait primaire pour obtenir une concentration de 5 à 25% en extrait sec, ceci afin d'éliminer les solvants miscibles avec l'eau. On extrait alors à l'aide d'un solvant ou d'un mélange de solvants du groupe I, de préférence de l'acétate d'éthyle.

Une fois effectuée la partition de l'extrait primaire (lequel a éventuellement subi les opérations préalables décrites ci-dessus), la phase alcool-eau est lavée avec du n-hexane ou de l'heptane, et de préférence avec de l'heptane, de sorte à éliminer les chlorophylles, stérols et autres lipides végétaux.

L'alcool de la phase alcool-eau est éliminé par évaporation et une nouvelle extraction de la phase aqueuse résultante est effectuée à l'aide d'acétate d'éthyle.

La phase acétate d'éthyle est ensuite séchée avec un agent desséchant tel que MgSO₄ ou Na₂SO₄ anhydre, filtrée et évaporée à sec.

L'extrait sec résultant de l'étape précédente peut être purifié par filtration, par exemple par filtration sur gel de silice en utilisant une charge comprise de 2 à 20%, de préférence d'environ 5%, en éluant avec des mélanges heptane-acétate d'éthyle de proportions comprises entre 8:2 et 1:1, de préférence 7:3, ou avec des mélanges toluène-acétone de proportions comprises entre 9:1 et 6:4, de préférence 7:3. Le filtrat est concentré à sec pour obtenir, selon l'invention, un extrait enrichi en mikanolide et dihydromikanolide.

Alternativement, l'extrait sec peut être purifié par cristallisation dans le méthanol, l'éthanol ou l'acétone, par exemple dans une une solution méthanolique comprenant une concentration en extrait sec de l'ordre de 10%, le mélange étant laissé reposer pendant une durée de préférence comprise entre 12 et 16 heures à une température de préférence comprise entre -10 et 5 °C. Après filtration (ou centrifugation), rinçage des cristaux avec un peu de méthanol et séchage, on obtient un extrait enrichi en mikanolide et dihydromikanolide.

Afin de convertir le mikanolide en dihydromikanolide, une étape d'hydrogénation peut être effectuée, par exemple en dissolvant l'extrait obtenu précédemment dans de l'acétate d'éthyle à une température de préférence comprise entre 10 et 35 °C en présence d'un catalyseur tel que Pd/CaCO₃ à 5% sous une pression de 1 à 2 atmosphères d'hydrogène. Cette étape d'hydrogénation présente par ailleurs l'avantage de faciliter la purification du mélange constitué par l'extrait enrichi en mikanolide et dihydromikanolide.

La cristallisation du dihydromikanolide à partir de la solution concentrée dans l'acétate d'éthyle est alors réalisée par addition d'un n-alcane liquide, par exemple de n-heptane ou de n-hexane, et de préférence de n-heptane. On obtient alors du dihydromikanolide à un degré de pureté au moins égal à 92%.

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus et ne doivent en aucun cas être considérés comme une limite à la portée de l'invention.

### EXEMPLES

### Exemple 1 :

100 g de feuilles sèches (contenant moins de 10% d'humidité) de *Mikania micrantha* (avec leurs tiges), récoltées entre mai et juillet dans la région autour de Sungei Ara, Penang (Malaisie) sont broyées dans un moulin pour donner des particules de taille inférieure à 4 mm. Après addition de 1 litre d'acétate d'éthyle, le mélange est agité vigoureusement pendant 30 minutes à une température de 55 à 60 °C. Le résidu solide est séparé par filtration et soumis à une seconde extraction dans les mêmes conditions. Les extraits des première et deuxième étapes d'extraction sont combinés. L'extrait est concentré sous pression réduite pour obtenir une concentration en contenu solide d'environ 5%, ce qui correspond à un volume d'environ 110 ml. On ajoute alors 40 ml de n-heptane.

125 ml d'un mélange méthanol-eau 1:2 est ajouté à l'extrait et agité vigoureusement dans une ampoule à décanter puis laissé décanter. La phase aqueuse inférieure est chauffée à environ 40-50 °C pour casser les émulsions éventuelles et faciliter la séparation de phases. La phase eau-méthanol est évacuée par le bas. La partition est répétée encore deux fois en utilisant à chaque fois 50 ml d'un mélange méthanol-eau 1:3.

20 ml de n-heptane sont alors ajoutés à la solution méthanol-eau. Après une agitation vigoureuse dans une ampoule à décanter, on laisse séparer les deux phases. La solution méthanol-eau dégraissée est ensuite évacuée par le bas.

La solution méthanol-eau est alors concentrée sous pression réduite pour éliminer le méthanol et obtenir une suspension aqueuse.

100 ml d'acétate d'éthyle et 15 g de chlorure de sodium sont ajoutés à la suspension aqueuse. Le mélange est agité vigoureusement dans une ampoule à décanter puis laissé décanter. L'extraction liquide-liquide est répétée une fois avec 50 ml d'acétate d'éthyle.

Les extraits acétate d'éthyle combinés sont séchés par addition d'environ 30 g MgSO₄, le mélange étant agité pendant environ 10-20 minutes avant d'être filtré (MgSO₄ étant rincé avec 2 fois 25 ml d'acétate d'éthyle). Le filtrat et l'acétate d'éthyle ayant servi au rinçage, combinés, sont ensuite évaporés à sec sous pression réduite.

L'extrait solide obtenu est ensuite dissous dans 5 ml MeOH et laissé cristalliser à 4 °C pendant 16 heures. On filtre alors sur fritté, les cristaux récupérés étant rincés avec 2 ml MeOH à 4 °C. Après séchage dans un four à vide, on obtient 481,2 mg d'extrait sec.

L'analyse par HPLC de l'extrait sec obtenu donne les résultats suivants :

| **Composé** | **Proportion en poids** |
|---|---|
| Mikanolide | 68,0 % |
| Dihydromikanolide | 25 % |
| Scandénolide | 5 % |

### Exemple 2 :

Le même processus que celui de l'exemple 1 est utilisé au début. Par contre, au lieu d'ajouter 5 ml MeOH après le séchage avec MgSO₄ et d'effectuer une cristallisation, on ajoute 2,8 g de célite et évapore à sec les solvants. On filtre alors sur un lit de silice (12 g SiO₂ 35-70 µm). On fait d'abord passer 4 fois 26 ml (soit quatre fois le volume de colonne) de mélange heptane / acétate d'éthyle 2:8 (les fractions correspondantes étant éliminées), puis 14 fois 26 ml de mélange heptane / acétate d'éthyle 6:4 (les fractions correspondantes étant récupérées). Les fractions récupérées sont combinées et évaporées à sec pour obtenir l'extrait sec final.

### Exemple 3 :

330 mg d'extrait sec tel qu'obtenu dans l'exemple 1 sont dissous dans 80 ml d'acétate d'éthyle et la solution, à laquelle sont ajoutés 248 mg de Pd/CaCO₃ à 5%, est placée sous flux d'hydrogène (pression comprise entre 1 et 2 atmosphères). L'agitation et le flux d'hydrogène sont maintenus pendant la nuit et l'achèvement de la réaction contrôlé par chromatographie HPLC. Une fois la réaction terminée, on ajoute 1 g de célite et on agite pendant 10 minutes. On ajoute ensuite 5 g de MgSO₄ anhydre et on élimine les solides par filtration sur célite. La célite est rincée avec 2 fois 10 ml d'acétate d'éthyle, puis les solvants éliminés par évaporation à sec. On ajoute 3 ml d'acétone puis 3 ml d'heptane au solide récupéré. On évapore partiellement les solvants au rotavapeur à 50°C puis on refroidit à 0 °C au moyen d'un bain de glace. Après filtration, on rince le solide avec 2 fois 1 ml d'un mélange 50/50 acétone/heptane. On évapore alors à sec et récupère 214 mg de dihydromikanolide.
RMN ¹H (DMSO d6, 400 MHz, δ): 0,96 (s, 3H); 1,25-1,27 (d, 3H); 1,84-1,89 (m, 1H) ; 2,04-2,10 (m, 1H); 2,49-2,50 (t, 1H); 2,93-2,98 (m, 1H); 3,19 (s, 1H); 3,38-3,39 (d, 1H); 3,98-3,99 (d, 1H); 4,62-4,68 (m, 1H); 5,42-5,43 (d, 1H); 7,61-7,62 (d, 1H). RMN ¹³C (DMSO d6, δ): 13,3 ; 20,9 ; 39,7 ; 41,1 ; 42,3 ; 50,3 ; 52,3 ; 54,9 ; 57,5 ; 76,7 ; 81,8 ; 128,7 ; 149,9 ; 171,1 ; 176,1.

### ETUDE PHARMACOLOGIQUE DES PRODUITS DE L'INVENTION

A titre d'exemple, on étudiera les effets d'un traitement par les composés de l'invention sur :
- l'incorporation de cytosine marquée au ³²P dans un ADN en présence d'ADN polymérase ( système acellulaire) ;
- l'incorporation de thymidine tritiée dans l'ADN des cellules tumorales en division durant un temps court (système cellulaire) ;
- la prolifération de deux lignées de cellules humaines Mia-Paca2 et DU145.

### 1) Procédures

### Lignées cellulaires

Les lignées cellulaires DU145 (cellules humaines de cancer de la prostate), HT29 (cancer du colon) et Mia-PaCa2 (cellules humaines de cancer du pancréas) ont été acquises auprès de American Tissue Culture Collection (Rockville, Maryland, USA).

### Incorporation de cytosine marquée au ³²P dans un ADN en présence d'ADN polymérase dans un système acellulaire

Le marquage de l'ADN se fait sur un fragment d'ADN qui incorpore des nucléotides grâce à l'activité de l'enzyme ADN polymérase. Parmi ces nucléotides, le dCTP est marqué par du phosphore radioactif (³²P).

L'ADN plasmidique (pc DNA 3, invitrogen, Pays-Bas) est dilué à une concentration de 2 ng dans 45 µl de solution TE (10mM Tris-HCl ; pH 8,0 ; 1mM EDTA) puis dénaturé par chauffage à 100 °C pendant 10min avant d'être replacé directement dans de la glace. L'ADN dénaturé est placé dans le tube qui contient la solution tampon de dATP, dGTP, dTTP, l'enzyme ADN polymérase Klenow sans exonuclease et les amorces aléatoires (Rediprime II random prime labelling system, RPN 1633, RPN 1634, Amersham pharmacia biotech). 2µl de Redivue [³²P]dCTP (1-déoxycytidine 5'(α-³²P), activité spécifique 250 µCi, Amersham), sont ajoutés pour démarrer la réaction. La réaction est faite en présence ou non du composé à tester durant 10 min à 37 °C ou 1 heure à température ambiante. La réaction est arrêtée par ajout de 5µl de EDTA à 0,2 M. Pour récupérer l'ADN, 200µl d'isopropanol sont ajoutés et le culot d'ADN est récupéré après 10 min de centrifugation à 12 000 tours/min puis est lavé dans l'éthanol à 70%. Après séchage complet à l'air ambiant, l'ADN est solubilisé dans 50 µl de TE. 10 µl sont comptés dans 5 ml de scintillant (Instagel® plus et compteur à scintillation Packard). Les résultats sont exprimés en pourcentage d'inhibition de l'incorporation du nucléotide marqué dans l'échantillon par rapport au témoin (100 - (DPM de l'échantillon/ DPM témoin)* 100).

### Incorporation de thymidine marquée au tritium dans l'ADN de cellules en phase exponentielle de croissance (in vitro)

Des cellules HT29 sont ensemencées dans des plaques 96 puits (culturPlate-96, Packard ; 4000 cellules par puits) avec le milieu (DMEM, Gibco BRL complémenté par 10% de sérum de veau foetal, Gibco BRL). Au jour 3, le milieu est enlevé et remplacé par du milieu contenant la thymidine tritiée (5'-thymidine TRK.328, 1 mCi, 0,6 µCi/puits, Amersham) avec ou sans les produits (gamme de 100 à 0,19 µg/ml par dilutions 1/2). Les traitements s'effectuent pendant 1 heure. Le milieu est alors enlevé et les cellules sont lavées 2 fois avec 200µl de PBS (Gibco BRL). La solution de lyse (SDS 1,25% ; EDTA 5 mM) est ajoutée et laissée pendant 10 min à température ambiante, puis 75 µl de liquide scintillant (microscint® 40, Packard) est ajouté. La lecture se fait sur Topcount® (Packard). Les résultats sont exprimés en pourcentage d'inhibition de l'incorporation du nucléotide marqué dans l'échantillon par rapport au témoin (100 - (DPM de l'échantillon / DPM témoin) * 100).

### Tests de prolifération cellulaire

Les cellules placées dans 80 µl de milieu Eagle modifié de Dulbecco (Gibco-Brl, Cergy-Pontoise, France) complété avec 10% de sérum foetal de veau inactivé par chauffage (Gibco-Brl, Cergy-Pontoise, France), 50000 unités/l de pénicilline et 50 mg/l streptomycine (Gibco-Brl, Cergy-Pontoise, France), et 2 mM de glutamine (Gibco-Brl, Cergy-Pontoise, France) ont été ensemencées sur une plaque de 96 puits au jour 0. Les cellules ont été traitées au jour 1 pendant 96 heures avec des concentrations croissantes de chacun des composés à tester jusqu'à 50 µg/ml. A la fin de cette période, la quantification de la prolifération cellulaire est évaluée par test colorimétrique en se basant sur le clivage du sel de tétrazolium WST1 par les déhydrogénases mitochondriales dans les cellules viables conduisant à la formation de formazan (Boehringer Mannheim, Meylan, France). Ces tests sont effectués en double avec 8 déterminations par concentration testée. Pour chaque composé à tester, les valeurs incluses dans la partie linéaire de la sigmoïde ont été retenues pour une analyse en régression linéaire et utilisées pour estimer la concentration inhibitrice CI₅₀. Les produits sont solubilisés dans le diméthylsulfoxide (DMSO) à 10⁻² M et utilisés en culture avec 0,5% DMSO en final.

### 2) Résultats :

Les résultats des tests d'inhibition de l'activité de l'ADN polymérase sont reportés dena le tableau I ci-après :

**Tableau I**

| *Inhibition de l'activité de l'ADN polymérase par le dihydromikanolide et le mikanolide (100 µg*/*ml) dans un système acellulaire* | |
|---|---|
| **Composé** | **Inhibition de l'incorporation (%)** |
| Dihydromikanolide | 73 (n = 5 ; écart-type σ = 7) |
| Mikanolide | 79,6 (n = 3 ; écart-type σ = 11,0) |

L'incorporation de la thymidine tritiée dans l'ADN des cellules humaines HT29 est inhibée quand ces dernières sont traitées par le dihydromikanolide et le mikanolide (cf. tableau II ci-après).

**Tableau II**

| **Inhibition de l'incorporation (dpm échantillon /dpm témoin)** | | | |
|---|---|---|---|
| Concentration (µg/ml) | 100 | 50 | 25 |
| Dihydromikanolide | 0,19 | 0,29 | 0,58 |
| Mikanolide | 0,53 | 0,61 | 1,04 |

Enfin, on trouvera dans le tableau III ci-après les valeurs *in vitro* des activités d'inhibition de la prolifération par rapport à des cellules tumorales humaines DU145 et Mia-Paca2 traitées par le mikanolide et les composés des exemples 1 et 2.

**Tableau III**

| *Inhibition de la prolifération des cellules tumorales humaines traitées par le mikanolide et les composés des exemples 1 et 2.* | | |
|---|---|---|
| **Composé** | **Concentration inhibitrice CI**_{**50**} **(µM)** | |
| | Mia-Paca2 | DU-145 |
| Mikanolide | 3 | 15 |
| Exemple 1 | 1,6 | 4,6 |
| Exemple 2 | 1,3 | 4,3 |

## Revendications

1. Procédé d'obtention d'un extrait sec de feuilles de *Mikania micrantha, Mikania scandens* et *Mikania cordata* comprenant au moins 50% en poids de mikanolide et de dihydromikanolide et au moins 20 % de dihydromikanolide, ledit procédé étant **caractérisé en ce qu'**il comporte les étapes successives suivantes :
- broyage et séchage de feuilles de *Mikania micrantha, Mikania scandens* et *Mikania cordata* ;
- addition, aux feuilles broyées de *Mikania micrantha, Mikania scandens* et *Mikania cordata*, d'un solvant choisi parmi le toluène, l'acétate d'éthyle, des mélanges de toluène et d'acétate d'éthyle, des mélanges de toluène et d'acétone, des mélanges d'acétate d'éthyle et d'heptane, et des mélanges d'heptane et d'acétone dans des proportions de 7:3 à 3:7 ;
- filtration et récupération du filtrat ;
- concentration de l'extrait de la solution d'extrait primaire pour obtenir une concentration de 2,5 à 10% en extrait sec ;
- partition de l'extrait concentré obtenu à l'étape précédente entre le solvant d'extraction et un mélange comprenant de 10% à 50% de méthanol ou d'éthanol dans l'eau ;
- lavage de la phase alcool-eau avec du n-hexane ou de l'heptane ;
- élimination de l'alcool de la phase alcool-eau ;
- extraction de la phase aqueuse obtenue avec de l'acétate d'éthyle ;
- séchage de la phase acétate d'éthyle avec un agent desséchant ;
- évaporation des solvants et récupération de l'extrait sec ;
- purification de l'extrait sec obtenu par une méthode de séparation physico-chimique ; et
- obtention, après élimination des solvants, de l'extrait sec souhaité.

2. Procédé selon la revendication 1, **caractérisé en ce** la partition de l'extrait concentré est réalisée à l'aide d'un mélange comprenant de 25 à 30% de méthanol dans l'eau.

3. Procédé selon la revendication 1 ou 2, **caractérisé en ce que** la méthode de séparation physico-chimique est une cristallisation dans le méthanol, l'éthanol ou l'acétone.

4. Procédé selon la revendication 1 ou 2, **caractérisé en ce que** la méthode de séparation physico-chimique est une filtration sur gel de silice.

5. Procédé de préparation, à partir d'un extrait sec de feuilles de *Mikania micrantha, Mikania scandens* et *Mikania cordata* comprenant au moins 50% en poids de mikanolide et de dihydromikanolide et au moins 20 % de dihydromikanolide, d'un extrait enrichi en dihydromikanolide, **caractérisé en ce qu'**il consiste en l'hydrogénation dudit extrait, dissous dans de l'acétate d'éthyle, à une température comprise entre 10 et 35 °C, en présence d'un catalyseur d'hydrogénation sous une pression de 1 à 2 atmosphères d'hydrogène.

6. Procédé de préparation du dihydromikanolide à partir d'un extrait de *Mikania micrantha, Mikania scandens* et *Mikania cordata* comprenant au moins 50% en poids de mikanolide et de dihydromikanolide et au moins 20 % de dihydromikanolide, **caractérisé en ce qu'**il comporte les étapes successives suivantes :
- hydrogénation dudit extrait au moyen d'un procédé selon la revendication 5 ;
- cristallisation du dihydromikanolide par ajout d'un n-alcane liquide à la solution dans l'acétate d'éthyle de l'extrait enrichi en dihydromikanolide et récupération dudit dihydromikanolide par filtration.

7. Utilisation :
- d'un extrait de *Mikania micrantha, Mikania scandens* et *Mikania cordata* comprenant au moins 50% en poids de mikanolide et de dihydromikanolide et au moins 20 % de dihydromikanolide, ou
- d'un extrait tel qu'obtenu par un procédé selon la revendication 5, ou
- du mikanolide, ou enfin
- du dihydromikanolide
pour préparer un médicament destiné à traiter les maladies prolifératives.

8. Utilisation selon la revendication 7, **caractérisée en ce que** le médicament préparé est destiné à traiter le cancer.

## Patentansprüche

1. Verfahren zur Herstellung eines Trockenextrakts von Blättern von *Mikania micrantha, Mikania scandens* und *Mikania cordata*, der mindestens 50 Gew.-% Mikanolid und Dihydromikanolid und mindestens 20 % Dihydromikanolid enthält, wobei dieses Verfahren **dadurch gekennzeichnet ist, daß** es die folgenden aufeinanderfolgenden Schritte umfaßt:
- Zerkleinerung und Trocknung von Blättern von *Mikania micrantha, Mikania scandens* und *Mikania cordata;*
- Versetzen der zerkleinerten Blätter von *Mikania micrantha, Mikania scandens* und *Mikania cordata* mit einem Lösungsmittel, das aus Toluol, Ethylacetat, Mischungen von Toluol und Ethylacetat, Mischungen von Toluol und Aceton, Mischungen von Ethylacetat und Heptan und Mischungen von Heptan und Aceton in Verhältnissen von 7:3 bis 3:7 ausgewählt ist;
- Filtration und Gewinnung des Filtrats;
- Konzentration des Extrakts der Primärextraktlösung, um eine Konzentration von 2,5 bis 10% Trockenextrakt zu erhalten;
- Aufteilen des im vorhergehenden Schritt erhaltenen konzentrierten Extrakts auf das Extraktionslösungsmittel und eine Mischung, die 10% bis 50% Methanol oder Ethanol in Wasser enthält;
- Waschen der Alkohol-Wasser-Phase mit n-Hexan oder Heptan;
- Entfernung des Akohols aus der Alkohol-Wasser-Phase;
- Extraktion der erhaltenen wässrigen Phase mit Ethylacetat;
- Trocknung der Ethylacetat-Phase mit einem Trocknungsmittel;
- Abdampfen der Lösungsmittel und Gewinnung des Trockenextrakts;
- Reinigung des erhaltenen Trockenextrakts mit Hilfe einer physikalisch-chemischen Trennmethode; und
- Erhalten des gewünschten Trockenextrakts nach Entfernung der Lösungsmittel.

2. Verfahren nach Anspruch 1, **dadurch gekennzeichnet, daß** die Aufteilung des konzentrierten Extrakts mit Hilfe einer Mischung vorgenommen wird, die 25 bis 30% Methanol in Wasser enthält.

3. Verfahren nach Anspruch 1 oder 2, **dadurch gekennzeichnet, daß** die physikalisch-chemische Trennmethode eine Kristallisation in Methanol, Ethanol oder Aceton ist.

4. Verfahren nach Anspruch 1 oder 2, **dadurch gekennzeichnet, daß** die physikalisch-chemische Trennmethode eine Filtration über Silikagel ist.

5. Verfahren zur Herstellung eines mit Dihydromikanolid angereicherten Extrakts aus einem Trockenextrakt von Blättern von *Mikania micrantha, Mikania scandens* und *Mikania cordata*, der mindestens 50 Gew.-% Mikanolid und Dihydromikanolid und mindestens 20 % Dihydromikanolid enthält, **dadurch gekennzeichnet, daß** es in der Hydrierung dieses Extrakts, der in Ethylacetat gelöst ist, bei einer Temperatur zwischen 10 und 35°C in Gegenwart eines Hydrierungskatalysators unter einem Druck von 1 bis 2 Athmosphären Wasserstoff besteht.

6. Verfahren zur Herstellung von Dihydromikanolid aus einem Extrakt von Blättern von *Mikania micrantha, Mikania scandens* und *Mikania cordata*, der mindestens 50 Gew.-% Mikanolid und Dihydromikanolid und mindestens 20 % Dihydromikanolid enthält, **dadurch gekennzeichnet, daß** es die folgenden aufeinanderfolgenden Schritte umfaßt:
- Hydrierung dieses Extrakts mit Hilfe eines Verfahrens nach Anspruch 5;
- Kristallisation des Dihydromikanolids durch Zusatz eines flüssigen n-Alkans zu der Lösung des mit Dihydromikanolid angereicherten Extrakts in Ethylacetat und Gewinnung des Diydromikanolids durch Filtration.

7. Verwendung
- eines Extrakts von *Mikania micrantha, Mikania scandens* und *Mikania cordata*, der mindestens 50 Gew.-% Mikanolid und Dihydromikanolid und mindestens 20% Dihydromikanolid enthält, oder
- eines mit Hilfe eines Verfahrens nach Anspruch 5 erhaltenen Extrakts oder
- von Mikanolid oder
- von Dihydromikanolid
für die Herstellung eines Medikaments für die Behandlung von proliferativen Krankheiten.

8. Verwendung nach Anspruch 7, **dadurch gekennzeichnet, daß** das hergestellte Medikament für die Behandlung von Krebs bestimmt ist.

## Claims

1. Process for obtaining a dry extract of *Mikania micrantha, Mikania scandens* and *Mikania cordata* leaves, comprising at least 50% by weight of mikanolide and dihydromikanolide and at least 20% dihydromikanolide, the said process being **characterised in that** it comprises the following successive stages:
- grinding and drying *Mikania micrantha, Mikania scandens* and *Mikania cordata* leaves;
- addition, to the ground leaves of *Mikania micrantha, Mikania scandens* and *Mikania cordata*, of a solvent chosen from toluene, ethyl acetate, toluene and ethyl acetate mixtures, toluene and acetone mixtures, ethyl acetate and heptane mixtures, and heptane and acetone mixtures in proportions of 7:3 to 3:7;
- filtration and recovery of the filtrate;
- concentration of the extract of the primary extract solution in order to obtain a concentration of 2.5 to 10% dry extract;
- dividing of the concentrated extract obtained in the preceding stage between the extraction solvent and a mixture comprising from 10% to 50% methanol or ethanol in water;
- washing of the alcohol-water phase with n-hexane or heptane;
- elimination of the alcohol of the alcohol-water phase;
- extraction of the aqueous phase obtained with ethyl acetate;
- drying of the ethyl acetate phase with a drying agent;
- evaporation of the solvents and recovery of the dry extract;
- purification of the dry extract obtained by a physico-chemical separation method; and
- obtaining, after elimination of the solvents, the desired dry extract.

2. Process according to claim 1, **characterized in that** the division of the concentrated extract is carried out using a mixture comprising from 25 to 30% methanol in water.

3. Process according to claim 1 or 2, **characterized in that** the physico-chemical separation method is crystallization from methanol, ethanol or acetone.

4. Process according to claim 1 or 2, **characterized in that** the physico-chemical separation method is filtration on silica gel.

5. Process for the preparation, from a dry extract of *Mikania micrantha, Mikania scandens* and *Mikania cordata* leaves comprising at least 50% by weight of mikanolide and dihydromikanolide and at least 20% dihydromikanolide, of a dihydromikanolide-enriched extract, **characterized in that** it consists of the hydrogenation of said extract, dissolved in ethyl acetate, at a temperature comprised between 10 and 35 °C, in the presence of a hydrogenation catalyst under a pressure of 1 to 2 hydrogen atmospheres.

6. Process for the preparation of dihydromikanolide from a *Mikania micrantha, Mikania scandens* and *Mikania cordata* extract comprising at least 50% by weight of mikanolide and dihydromikanolide and at least 20% dihydromikanolide **characterized in that** it comprises the following successive stages:
- hydrogenation of said extract by means of a process according to claim 5;
- crystallization of the dihydromikanolide by adding a liquid n-alkane to the solution in ethyl acetate of the dihydromikanolide-enriched extract and recovery of said dihydromikanolide by filtration.

7. Use:
- of a *Mikania micrantha, Mikania scandens* and *Mikania cordata* extract comprising at least 50% by weight of mikanolide and dihydromikanolide and at least 20% dihydromikanolide, or
- of an extract as obtained by a process according to claim 5, or
- of mikanolide, or finally
- of dihydromikanolide
to prepare a medicament intended for the treatment of proliferative diseases.

8. Use according to claim 7, **characterized in that** the medicament prepared is intended for the treatment of cancer.
